# EUROPEAN PATENT APPLICATION

(11) **EP 3 363 398 A1**
(43) Date of publication of application: **22.08.2018**
(21) Application number: 18275022.4
(22) Date of filing: 15.02.2018
(51) Int. Cl.: A61B 18/14, A61B 18/00

(54) **CUTTING SYSTEM FOR MEDICAL TREATMENT**

(30) Priority: 15.02.2017 US 201762459344 P
(71) Applicant: Cook Medical Technologies LLC, Bloomington, IN 47404 (US)
(72) Inventor: ROEDER, Blayne A., Bloomington, Indiana 47401 (US); ROEDER, Rebecca A., Bloomington, Indiana 47401 (US); CHAMBERS, Sean E., Bloomington, Indiana 47401 (US); NOBLET, Jillian, Bloomington, Indiana 47404 (US); BERWICK, Zachery, Costa Mesa, California 92626 (US); PHILLIPS, Matthew J., Carlsbad, California 92009 (US); KASSAB, Ghassan, La Jolla, California 92037 (US); KRIEGER, Joshua F., Topsfield, Massachusetts 01983 (US); KRATZBERG, Jarin, Lafayette, Indiana 47909 (US)
(74) Representative: Williams Powell

(57) **Abstract**

A tissue cutting system (10) includes a first device (30) and a second device (50) adapted for coupling to a power supply (12), the first device including a conductive probe (38, 70) and the second device including a conductive material (58, 104), for example, as part of an expandable structure (100), having an outer surface area of conductive material that is larger than a surface area of the conductive probe. In use, the conductive probe and the conductive material are deployed along opposite sides of a body tissue, such as an aortic dissection flap, and selective electrical power is applied to the conductive probe and the conductive material to cut the body tissue.

## Description

### BACKGROUND

The present disclosure relates generally to medical devices, systems, and methods, and particularly, to systems and techniques for selectively cutting tissue walls for medical treatment, such as, for example, for treatment of aortic dissection.

An aortic dissection is a condition that may occur, for example, to the descending aorta (referred to as Type B dissections) in which the wall of the aorta is damaged to such an extent that blood under pressure can get between inner and outer layers of the wall of the aorta to expand part of the wall into an inflated sac of blood which is referred to as a false lumen (e.g., the aortic dissection may be caused by an aneurysm). The inflated sac of blood or false lumen so formed may extend some distance down the descending aorta and open out into the aorta again further down. In the acute phase, dissections may close off perfusion from the aorta to vital organs. In the chronic phase, the weakened tissue can develop into aneurysm and ultimately rupture. Dissections involving the ascending aorta are referred to as Type A dissections.

Several treatments have been explored, including monitoring to lower the blood pressure of the patient and reducing the hemodynamic stresses on the diseased vessel, and for some patients, performing surgery. Portions of the diseased aorta may be replaced by a graft for reattachment of the dissection flap. An open repair may involve the replacement of the entire dissected segment with a graft. Stent grafts have been used in the true lumen to reappose the true lumen with the goal to thrombose the false lumen and maintain patency of the true lumen. However, it may be difficult to reappose the true lumen in the treatment of acute dissections due to contraction of the intima and/or expansion of the media and adventitia in the acute setting.

There is a clear need for an improved method to treat aortic dissections. The current application provides novel solutions to the treatment of aortic dissections.

### SUMMARY

Tissue cutting systems and methods of cutting a tissue are provided. In one example, the tissue cutting system includes a first device and a second device. The first device includes a first outer sheath with a first outer sheath lumen disposed about a first device axis, and a conductive probe slidably disposed within the first outer sheath lumen. The conductive probe includes an electrically operable tip. In a deployed configuration, the tip is disposed outside the first outer sheath lumen and axially movable away the first device axis. The second device includes an electrically operable conductive material of an outer surface area. The outer surface area is larger than a tip surface area of the tip. In response to electrical power provided to at least one of the conductive probe and the conductive material, the tip of the first device and the conductive material of the second device are operable to generate a RF arc.

In another example, an electrosurgical tissue cutting system includes first and second devices. The first device includes a first outer sheath defining a sheath lumen about a first device axis, and an active electrode slidably disposed within the sheath lumen of the first outer sheath. The active electrode includes an electrically conductive tip. The active electrode has a delivery configuration and a deployed configuration. When in the deployed configuration, the electrically conductive tip is radially movable away the first device axis outside the first outer sheath. The second device includes a return electrode, and the return electrode includes a radially expandable outer surface with a conductive material. The active and return electrodes are operable to generate a RF arc in response to being coupled to electrical power from a power supply.

In another example, the method of cutting a tissue includes one or more of the following steps in any order. A step includes inserting a first device in a first vessel lumen of a body vessel. The first device includes an axially movable conductive probe, and the axially movable conductive probe includes an energizable tip. A step includes inserting a second device in a second vessel lumen of the body vessel. The second device includes a conductive material. A step includes positioning the conductive material of the second device within the second vessel lumen against a body wall tissue at a point of treatment. A step includes positioning the energizable tip of the first device within the first vessel lumen against the body wall tissue at the point of treatment obverse to the conductive material. A step includes selectively energizing the energizable tip of the first device and the conductive material of the second device, thereby at least partially cutting through the body wall tissue at the point of treatment.

Other systems, methods, features and advantages of the disclosed features will be, or will become, apparent to one with skill in the art upon examination of the following figures and detailed description. It is intended that all such additional systems, methods, features and advantages be within the scope of this disclosure, and be encompassed by the following claims.

### BRIEF DESCRIPTION OF THE DRAWINGS

The features may be better understood with reference to the following drawings and description. The components in the figures are not necessarily to scale, emphasis instead being placed upon illustrating the principles of this disclosure. Moreover, in the figures, like referenced numerals designate corresponding parts throughout the different views.
FIG. 1 shows an example of a tissue cutting system.
FIG. 2 shows a distal end of a first device of the tissue cutting system of FIG. 1.
FIG. 3 shows a side view of a distal end of an example of a deployed conductive probe of a first device of the tissue cutting system of FIG. 1.
FIG. 4 shows a bottom view of a distal end of an example of a deployed conductive probe of a first device of the tissue cutting system of FIG. 1.
FIG. 5 shows a bottom view of a distal end of an example of a deployed conductive probe of a first device of the tissue cutting system of FIG. 1, depicting the conductive probe as retrievable.
FIG. 6 shows a balloon device embodiment of the conductive probe of the first device.
FIG. 7 shows a distal end of an example of a radially expandable outer surface of a second device of the tissue cutting system of FIG. 1.
FIG. 8 shows the distal end of the radially expandable outer surface of FIG. 6 as retrievable.
FIG. 9 shows a distal end of another example of a radially expandable outer surface of a second device of the tissue cutting system of FIG. 1, in the form a balloon device.
FIG. 10 shows an exemplary first desired pattern of electrically conductive areas on the first device.
FIG. 11 shows an exemplary second desired pattern of electrically conductive areas on the second device.
FIG. 12 shows an exemplary pattern of cuts made to a tissue by the tissue cutting system.
FIG. 13 shows the tissue cutting system implementing an exemplary step in a method for cutting a tissue.
FIG. 14 shows the tissue cutting system implementing an exemplary step in a method for cutting a tissue.
FIG. 15 shows the tissue cutting system implementing an exemplary step in the method for cutting a tissue.
FIG. 16 shows the tissue cutting system implementing an exemplary step in the method for cutting a tissue.
FIG. 17 shows the tissue cutting system implementing an exemplary step in the method for cutting a tissue.
FIG. 18 shows the tissue cutting system implementing an exemplary step in the method for cutting a tissue.
FIG. 19 shows a pattern of cuts formed in a tissue as a result of the method of cutting a tissue using an example of the tissue cutting system.
FIG. 20 shows another embodiment where the first device includes the balloon device.
FIG. 21A shows an expansion of a true lumen with a subsequent medical device after usage of the tissue cutting device.
FIG. 21B shows an expansion of a true lumen with a subsequent medical device after usage of the tissue cutting device.
FIG. 22 shows an exemplary pattern of cuts made to a tissue by the tissue cutting system.

### DETAILED DESCRIPTION OF THE PREFERRED EMBODIMENTS

Electrosurgical tissue cutting systems and methods for tissue treatment are described herein. The system includes first and second devices having cutting devices, which function as active and return electrodes, such as, for example, in a bipolar, sesquipolar or other arrangement. When the devices are located against the obverse sides of the tissue, such as a dissection flap, and energized, the tissue is at least partially cut in-situ or in-vivo into the intima in order to reduce the tissue's resistance to displacement or to alleviate the circumferential strength of the intima. In some instances, one or more cuts, such as for example, openings or slits, are formed in the tissue. In the case of partial cuts or full opening cuts, it may be particularly beneficial in reapposing the dissection flap to the aortic wall, which has been difficult likely due to contraction of the intima and/or expansion of the media and adventitia in the acute setting. That is, after a suitable number of cuts are at least partially formed into the dissection flap and the devices are removed from the body, other devices, such as but not limited to, stents, stent grafts, or balloons, may be inserted and located in the true lumen of the vessel at the cuts and expanded to reappose the dissection flap to the aortic wall. The term "cuts" is used herein to describe partial tissue cuts where a partial portion of tissue is removed and full tissue cuts where tissue is removed fully to define an opening therethrough.

In the present application, the term "distal" when referring to a delivery device refers to a direction that is farthest away from an operator using a delivery device, while the term "proximal" refers to a direction that is generally closest to the operator using the delivery device. The distal and proximal ends of a delivery device may also be referred to as an introduction end of the delivery device and an operator end of the delivery device, respectively. The term "operator end" of the delivery device is that portion of the device that is intended to remain outside of a patient during a procedure. The term "introduction end" of the delivery device, which is opposite to the operator end, is that portion of the device that is intended to be inserted within a patient during a procedure.

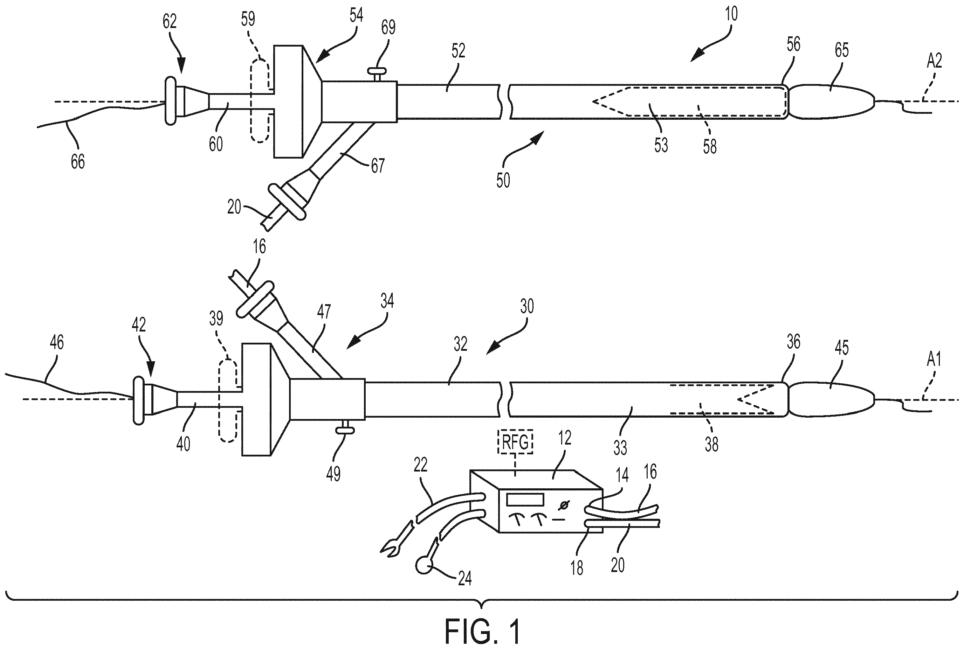

**FIG. 1** depicts one example of an electrosurgical tissue cutting system 10. The cutting system 10 may include a power supply 12 in electrical communication with a first device 30 and a second device 50. For example, a connector end 14 of a first electrical conductor 16 may electrically connect the first device 30 to one pole of a radio frequency (RF) generator RFG housed in the power supply 12 by, for example, a banana connector. A connector end 18 of a second electrical conductor 20 may electrically connect the second device 50 to the other pole of the RF generator of the power supply 12 by, for example, another banana connector. The power supply 12 may include a power conductor 22 for providing power to the power supply 12. The power supply 12 may be any device for supplying electrical power or current to the first device 30 and the second device 50. A control switch 24 may be included with the power supply 12 to provide selective electrical power or current from the power supply to the first device 30 and the second device 50. Manipulation of the control switch 24 may vary the delivery of power from a maximum level to some fraction of maximum level. The control switch 24 may be any device, such as but not limited to, a foot pedal, a button, a rotary dial, a slider switch, or a digital input.

The first device 30 may include a first outer sheath 32 with a tubular body 33 extending between a proximal end 34 and a distal end 36 and disposed about a first device longitudinal axis A1. The first outer sheath 32 is movable in the distal and proximal directions for selective deployment of a first cutting device 38 (in dashed lines) which is housed within a longitudinal sheath lumen of the first outer sheath 32, as will be described. An inner cannula 40 may be disposed within the sheath lumen of the first outer sheath 32. In one example, the first cutting device 38 may be disposed within a longitudinal annular lumen defined between the first outer sheath 32 and the inner cannula 40. The first cutting device 38 may be coupled to the inner cannula 40. The inner cannula 40 may be a tubular body extending between a proximal end 42 and a distal end (not shown). The distal end 36 may be coupled to a nose cone dilator 45. A first guidewire 46 may be inserted within a cannula lumen of the inner cannula 40. In one example, the distal end 36 may include a steerable tip or may have a curvature (not shown) away from the first device longitudinal axis A1, such as at a gradual radius, to place the distal end 36 about 20 degrees from the axis A1 to accommodate an elongate shaped conductor probe without a curvilinear portion.

In another example, a second elongated tube (not shown) may extend within the sheath lumen of the first outer sheath 32, having a distal end coupled to the first cutting device 38. The second elongated tube (not shown) may extend over the inner cannula 40 when employed. A proximal end 39 of the second elongated tube (shown in dashed lines) may extend beyond the proximal end 34 of the first outer sheath 32 so that the operator may have access to it for manipulation of the first cutting device 38 independent of the first outer sheath 32 and the inner cannula 40. Additional locking devices may be used to temporarily fix the position of the second elongated tube and thus the first cutting device 38 relative to the first outer sheath 32 and the inner cannula 40.

The first outer sheath 32 may also include a side branch 47 (as shown) or other port for receiving the first electrical conductor 16. The side branch 47 defines a branch lumen in communication with the sheath lumen of the first outer sheath 32. The first electrical conductor 16 is shown extending within the branch lumen of the side branch 47 and farther within the sheath lumen for electrical connection to the first cutting device 38. The position of the first outer sheath 32 relative to the inner cannula 40 is adjustable and then lockable to a positon by a locking mechanism 49, such as for example a rotatable vice pin or locking collar.

The second device 50 may comprise a second outer sheath 52 with a tubular body 53 extending between a proximal end 54 and a distal end 56 disposed about a second device longitudinal axis A2. The second outer sheath 52 is movable in the distal and proximal directions for selective deployment of a second cutting device 58 which may be housed within a longitudinal sheath lumen of the second outer sheath 52, as will be described. An inner cannula 60 of the second device 50 may be disposed within the sheath lumen of the second outer sheath 52. In one example, the second cutting device 58 may disposed within a longitudinal annular lumen defined between the second outer sheath 52 and the inner cannula 60. The second cutting device 58 may be coupled to the inner cannula 60. The inner cannula 60 may be a tubular body extending between a proximal end 62 and a distal end (not shown). The distal end may be coupled to a nose cone dilator 65. A second guidewire 66 may be inserted within a cannula lumen of the inner cannula 60.

In another example, a third elongated tube (not shown) may extend within the sheath lumen of the second outer sheath 52, having a distal end coupled to the second cutting device 58. The third elongated tube (not shown) may extend over the inner cannula 60 when employed. A proximal end 59 of the third elongated tube (shown in dashed lines) may extend beyond the proximal end 54 of the second outer sheath 52 so that the operator may have access to it for manipulation of the second cutting device independent of the second outer sheath 52 and the inner cannula 60. Additional locking devices may be used to temporarily fix the position of the third elongated tube and thus the second cutting device 58 relative to the first outer sheath 32 and the inner cannula 40.

The second outer sheath 52 may also include a side branch 67. The side branch 67 defines a branch lumen in communication with the sheath lumen of the second outer sheath 52. The second electrical conductor 20 is shown extending within the branch lumen and farther within the sheath lumen for electrical connection to the second cutting device 58. The position of the second outer sheath 52 relative to the inner cannula 60 is adjustable and then lockable to a positon by a locking mechanism 69, such as for example a rotatable vice pin or locking collar.

The above-described components of the first and second devices may include sealing devices disposed at relevant portions of the components to prevent fluid or blood loss. As may be appreciated by one of ordinary skill in the art, the electrical conductors, guidewires, inner cannulas, and optional elongated tubes may be extended in a sealing manner. As known, the various ports of the outer sheaths and the optional elongated tubes through which the components are passed through may provide a sealing surface of the sealing devices, such that the components may be sealed around and which will prevent loss of blood. The sealing devices and surfaces may be made of silicone or a similar material.

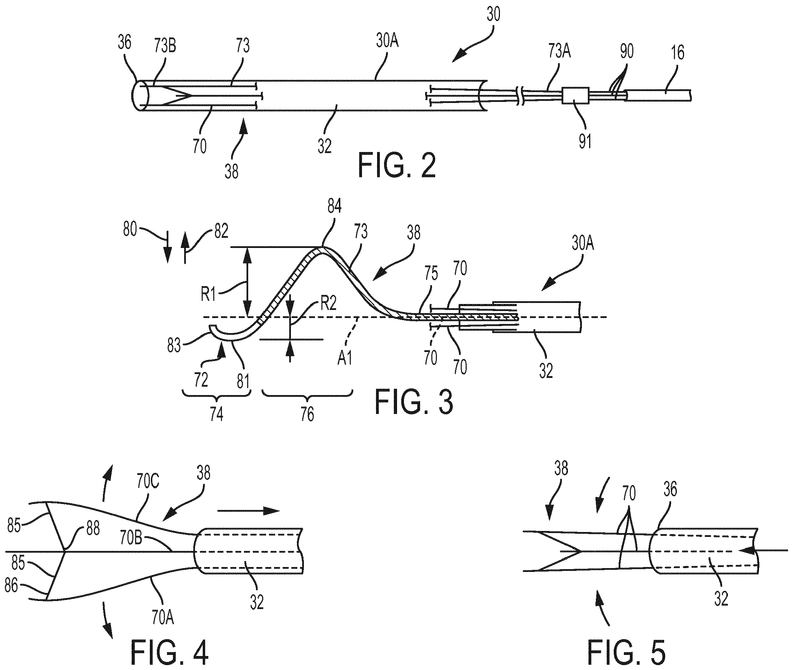

**FIG. 2** depicts a partial cutaway of a distal region 30A of the first device 30, with the first outer sheath 32 in its extended position over the first cutting device 38 in its delivery configuration. A portion of the first outer sheath 32 is shown removed to illustrate the coupling between the first electrical conductor 16 and the first cutting device 38, as will be described. **FIG. 3** depicts the distal region 30A of the first device 30, with the first outer sheath 32 in its retracted position and the first cutting device 38 in its deployed configuration. The first outer sheath 32 may be made of an insulating jacket material or have an inner lumen surface coating of an insulating material.

**FIGS. 3-4** also depict an example of the first cutting device 38 according to a first embodiment where the first cutting device 38 is comprised of one or more conductive probes 70 each having a respective energizable tip 72. In **FIG. 4**, three conductive probes 70A, 70B, 70C are shown, although it may be appreciated that less or more than three conductive probes may be used. The conductive probe 70 is axially movable between a deployed configuration (**FIG. 3**) and a delivery configuration (**FIG. 2**). In the delivery configuration, the conductive probe 70 is disposed within the sheath lumen of the first outer sheath 32. In the deployed configuration, the conductive probe 70 is disposed beyond the distal end 36 of the first outer sheath 32. Reference to the energizable tip 72 may include reference to a plurality of energizable tips.

Each of the conductive probes 70 includes an elongated body 73 longitudinally extending between a proximal end 73A and a distal end 73B. The elongated body 73 may include an electrical conductive material. An insulating jacket 75 may be disposed about the elongated body 73, leaving the energizable tip 72 exposed. In one example, the elongated body 73 has a curvilinear shaped portion proximate the distal end 73B. The energizable tip 72 is shown disposed along the curvilinear shaped portion of the elongated body 73. In another example, the curvilinear shaped portion of the elongated body 73 includes a first curved region 74 and a second curved region 76 disposed proximal to the first curved region 74. Here, the first curved region 74 includes the energizable tip 72. The first curved region 74 may be disposed radially from the first device longitudinal axis A1 in a first radial direction 80 by a first radial distance R1. The second curved region 76 may be disposed radially from the first device longitudinal axis A1 in a second radial direction 82 by a second radial distance R2. The second radial direction 82 may be different from the first radial direction 80. In one example, the first and second radial directions 80, 82 extend in directions that are about 150 degrees to 210 degrees apart, and, in one example, about 180 degrees apart. The second radial distance R2 may be greater than the first radial distance R1.

As shown in **FIG. 3**, the energizable tip 72 includes an engagement region 81 disposed at a curved bend 83 of the first curved region 74. Instead of the curved bend 83, the engagement region 81 may be disposed at an angular bend or corner or formed along a planar region. The curvature of the curved bend 83 is disposed radially outward from the first device longitudinal axis A1 such that the engagement region 81 faces radially away from the first device longitudinal axis A1. The engagement region 81 of the energizable tip 72 defines a tip surface area of electrical conductive material. In another example, the elongated body 73 may include a zigzag portion in addition to or instead of a curved region described above. A corner formed by the zigzag portion body is where the energizable tip 72 is located. The energizable tip 72 may also be disposed along a linear portion or other geometric shape portions to define a desired cut shape. The second curved region 76 is shown with an atraumatic bend 84. The atraumatic bend 84 is configured to engage the tissue wall of the vessel.

The conductive probe 70 may be spring biased to the curvilinear shaped portion such that when in the deployed configuration and the first outer sheath 32 is removed in the proximal direction relative to the first cutting device 38, the curvilinear shaped portion radially expands under its own resilience, as shown by the arrows in **FIG. 4****.** The conductive probe 70 may remain outside the first outer sheath 32 and repositioned for additional cutting procedures. In addition, the conductive probe 70 may be retrievable, as shown in **FIG. 5**, within the first outer sheath 32 either for repositioning of the conductive probe 70 or for complete removal from the body vessel. To this end, the curvilinear shaped portion of the conductive probe 70 is radially contractible as shown by the arrows from its radially expanded configuration when the first outer sheath is moved distally (as shown by the arrow) relative to the first cutting device 38. This not only allows the conductive probe to fit within the sheath lumen of the first outer sheath 32 in the delivery configuration, but also retrievable within the first outer sheath when moved over the expanded curvilinear shaped portion.

When more than one conductive probe 70 is used, the extension length of each of the conductive probes may be about the same length to create a cut pattern of aligned cuts. In other examples, the extension length of each of the conductive probes may differ to create a cut pattern of alternating cuts. The energizable tips of the conductive probes may be positioned to form an arcuate pattern (that is, where the middle tip is higher in elevation than the outside ones) or a planar pattern (that is, where all the tips are at the same elevation).

Connector members 85 may be coupled in between adjacent conductive probes (probes 70A and 70B and probes 70B and 70C) to fix the circumferential spacing between the conductive probes when separated in the deployed configuration. The connector member 85 may include a wire body or a strut body made of a flexible biocompatible material, and in some instance, an insulated material. The connector member 85 may include a single body (as shown) or may be made up of an assembly of pieces. The connector member 85 is collapsible or otherwise movable to allow for the radial contraction of the conductive probes 70 when housed in the delivery configuration within the first outer sheath 32. As shown, a first end 86 of the connector member 85 is coupled to one of the conductive probes 70 and its second end 88 is coupled to the second conductive probe. In one example, the connector member is coupled between the energizable tips. In one example, the connector member 85 is coupled between adjacent first curved regions of the conductive probes.

A portion of the first outer sheath 32 is shown removed to illustrate the coupling between the first electrical conductor 16 and the first cutting device 38. The first electrical conductor 16 may include the appropriate number of wires 90 for transmitting the electrical energy to the elongated body 73 of the conductive probes 70. The electrical connection may allow individual energization of the conductive probes 70. In one example, the conductive probes 70 are coupled to the wires 90 via soldering or wire coupling devices. In some examples, the probes 70 are extensions of the wires 90 within the sheath of the first electrical conductor. A terminal connector 91 may be used to electrically connect the conductive probes 70 and the wires 90 of the first electrical conductor 16. In one example, the terminal connector 91 may have individual terminations suitably configured to communicate electrical energy to the conductive probes 70 made of, for example, Nitinol or other materials, from wires 90 made of, for example, copper or other materials, of the first electrical conductor.

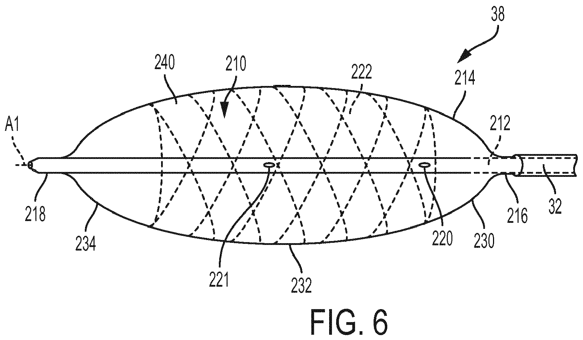

**FIG. 6** depicts an example of the first cutting device 38 according to a second embodiment where the first cutting device 38 is comprised of an inflatable balloon device 210. An inner cannula 212 of the balloon device 210 is surrounded by a balloon membrane 214 sealably attached to the inner cannula 212 at proximal and distal attachment locations 216, 218. The inner cannula 212 includes one or more longitudinal inflation lumens having a proximal end (not shown) that is capable of communication with a syringe of inflation fluid or the fluid delivery device. The distal end of the inflation lumen is in communication with ports 220, 221 formed into the sidewall of the inner cannula 212. Inflation fluid may be introduced at the proximal end of the first cutting device 38, traversing the inflation lumen and exiting the ports 220, 221 into an inflation annular lumen 222 defined between the inner surface of the balloon membrane 214 and the outer surface of the inner cannula 212 covered and surrounded by the balloon membrane 214. Continued inflation at a suitable pressure causes the balloon membrane 214 to radially expand. In the radially expanded configuration, the balloon membrane 214 may be shaped to have a proximal tapered section 230, a working body section 232, and a distal tapered section 234. The inflation fluid may be an electrically non-conductive to inhibit electrical energy flow into the first device outside the conductive surface. The working body section 232 may define a cylindrical shape as shown or may be tapered in a flatter, paddle shape, for better conformity with the body vessel. Removal of the inflation fluid from the inflation annular lumen 222 at the proximal end of the first cutting device 38 via the inflation lumen causes the balloon membrane 214 to deflate and radially compress into a smaller profile. The first outer sheath 32 may be included for selectively sliding over the balloon membrane 214 when in the deflated configuration.

A sleeve 240 (shown in dashed lines) may be disposed about the outer surface of the working body section 232 of the balloon device 210. For the working body section 232, the sleeve may wrap around the outside of the working body section 232 and be attached thereto, for example, by an adhesive or other suitable attachment means. The sleeve 240 may comprise of a solid sheet of electrical conductive material or a perforated or otherwise slotted sheet of electrically conductive material in a desired pattern. Alternatively, the sleeve 240 may comprise a solid sheet of electrically conductive material covered by an electrically insulating material, where the electrically insulating material is selectively removed to expose the electrically conductive material in a desired pattern. Alternatively, the sleeve 240 may comprise a solid, perforated, or otherwise slotted polymer or textile sheet attached to the working body section 232 and having an electrically conductive material disposed thereon in a desired pattern. In one example, a pattern of electrically conductive material may be printed or otherwise applied to the outer surface of the working body section 232 of the balloon membrane 214, or to the outer surface and/or inner surface of the sleeve 240 when employed.

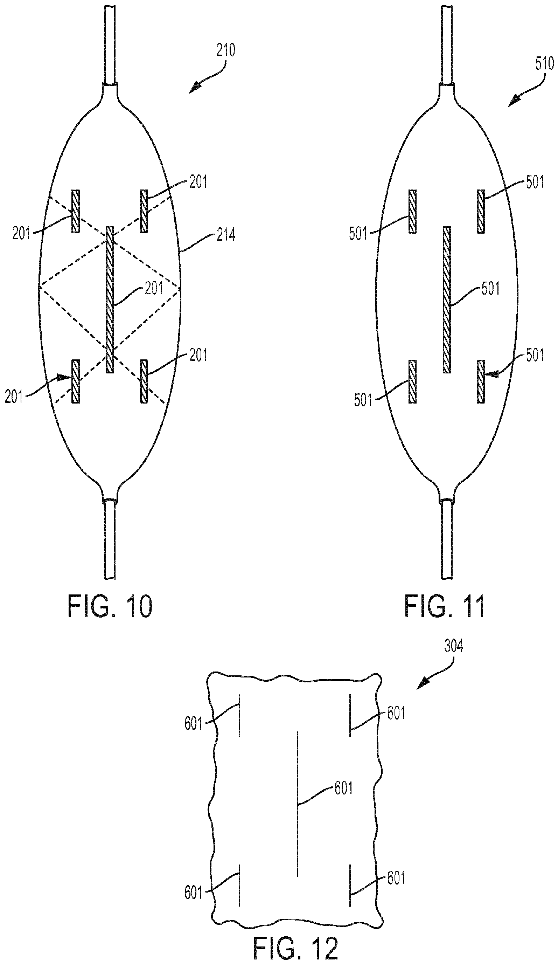

According to some embodiments, in addition to or instead of the sleeve 240, the material of the balloon membrane may be comprised of an electrically conductive material, such as a polymer interspersed with the electrically conductive material to create, according to some embodiments, a desired pattern of electrically conductive areas. For the working body section 232, the sleeve 240 may wrap around the outside of the working body section 232 and attach thereto, for example, by an adhesive or soldered. In addition to the crisscross pattern shown by the sleeve 240 in **FIG. 6**, **FIG.10** shows another exemplary desired pattern comprised of electrically conductive areas 201 exposed on the balloon device 210. The desired pattern shown in **FIG. 10** includes five distinct electrically conductive areas 201 in the desired pattern. As described, the desired pattern of electrically conductive areas 201 may be exposed areas on the sleeve 240, or alternatively printed or otherwise applied to the outer surface of the working body section 232 of the balloon membrane 214, or to the outer surface and/or inner surface of the sleeve 240. Other desired patterns of electrically conductive areas 201 are within the scope of this disclosure.

The electrically conductive material that is applied or printed may be a flowable ink material. The electrically conductive material may be printed on the sleeve 240 or directly on the working body section 232 of the balloon device 210 (e.g., balloon membrane 214) using pad printing or needle ink printing. Factors for selecting suitable electrically conductive materials are the flexibility of the material comprising the balloon membrane 214 and the flexibility of the printed electrically conductive material. The electrically conductive material may be applied to the balloon membrane 214 when the balloon device 210 is in the deflated configuration or the fully or partially inflated configuration.

When more than one desired pattern is included on the balloon device 210, a same desired pattern, or at least two different desired patterns, may be repeated around a circumference of the balloon device 210 (e.g., around a circumference of the working body section 232). Alternatively, when more than one desired pattern is included on the balloon device 210, a same desired pattern, or at least two different desired patterns, may be repeated across multiple locations on the balloon device 210 (e.g., repeated across area of the working body section 232). It follows that the placement of the desired pattern on the balloon device 210 may be a combination of any known pattern of one or more desired patterns.

The first electrical conductor 16 may include the appropriate number of wires for transmitting the electrical energy to the balloon device 210 with the electrically conductive material of the balloon device 210. The first electrical conductor 16, or an intermediate conductor connecting the first electrical conductor 16 to the balloon device 210, may be extended within the first outer sheath 32 and having a contact end for electrically coupling to the electrically conductive material of the balloon device 210 at one or more locations of the balloon device 210. This way, the power supply 12 is able to control power transmitted to the balloon device 210 to electrically energize each of the electrically conductive areas that comprise the desired pattern formed by the electrically conductive material together at a same time, or control power to each of the electrically conductive areas that comprise the desired pattern separately and individually. When more than one desired pattern is included on the balloon device 210 (e.g., same desired pattern repeated two or more times, or at least two different desired patterns), the power supply 12 is able to control power transmitted to each of the desired patterns together, or individually.

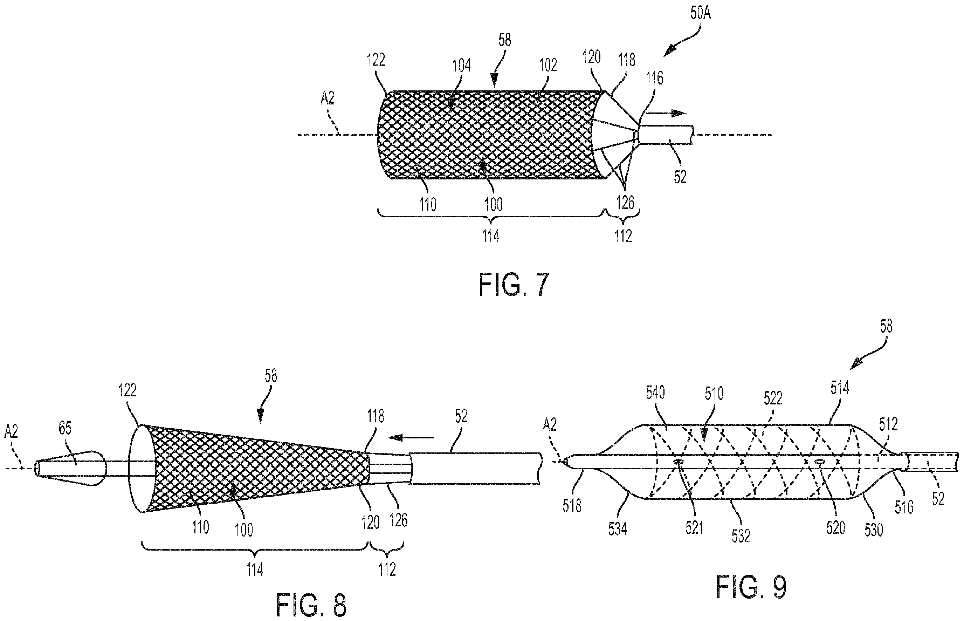

**FIG. 7** depicts one example of the second cutting device 58 disposed at a distal region 50A of the second device 50. The second cutting device 58 is shown having an expandable body 100 that includes an outer surface 102 with an electrically conductive material 104. The expandable body 100 is movable between a radially contracted configuration (**FIG. 1**), and a radially expanded configuration (**FIG. 7**). In particular, **FIG. 7** depicts the second outer sheath 52 in its retracted position and the second cutting device 58 in its radially expanded configuration. The amount of the electrically conductive material 104 defines an outer surface area when the second cutting device 58 is in its radially expanded configuration. The outer surface area of the expandable body 100 comprising the second cutting device 58 may be at least greater than the tip surface area of the energizable tip 72 comprising the first cutting device 38 according to some embodiments. The outer surface area of the expandable body 100 comprising the second cutting device 58 may also be at least greater than the surface area of the balloon device 210 comprising the first cutting device 38 according to some embodiments. In one example, the ratio between the outer surface area of the expandable body 100 to the surface area of the energizable tip 72, as well as the ratio between the outer surface area of the expandable body 100 to the surface area of the balloon device 210, defines a sesquipolar electrode arrangement.

The expandable body 100 may be coupled to the inner cannula 60 of the second device. Alternatively, the expandable body 100 may be coupled to the third elongated tube, as described above. The second outer sheath 52 is sized and configured for repetitive movement over and away from the expandable body 100 and relative to the third elongated tube when employed. For example, the second outer sheath 52 is slidably movable over the expandable body 100 to radially compress the expandable body 100 and away from the expandable body 100 for its radial expansion. **FIG. 8** depicts the second outer sheath 52 moving relative to the second cutting device 58, in the arrow shown, to radially compress the second cutting device 58.

According to **FIG. 7**, the expandable body 100 may be defined by a metal frame 110 comprised of at least a proximal tapered section 112 and a body or barrel section 114 distal to the proximal tapered section 112. A proximal end 116 of the proximal tapered section 112 is coupled to the inner cannula 60 or the distal end of the third elongated tube when employed. A distal end 118 of the proximal tapered section 112 is coupled to a proximal end 120 of the body section 114. The body section 114 extends axially about the second device longitudinal axis A2 between the proximal end 120 and its distal end 122. The proximal end 116 of the proximal tapered section 112 maintains its cross-sectional area during radial expansion of the body section 114, while the distal end 118 expands radially outward to a larger cross-sectional area during radial expansion of the body section.

The proximal tapered section 112 and the body section 114 may have many configurations in order to allow for movement between radial expansion and compression and the electrical communication with the energizable tip 72. The proximal tapered section may comprise of wire members 126 or a mesh wire frame (not shown). For example, the wire members 126 are circumferentially spaced from one another and taper outwardly relative to the second device longitudinal axis A2 during and at full radial expansion. Although **FIG. 7** depicts one example of the second cutting device 58 having an expandable body 100, in other embodiments the second cutting device 58 disposed into a true lumen area may take the form of conductive wires, the same as that comprise the first cutting device 38 described herein. For example, the second cutting device 58 may be comprised of an opposing set of conductive wires to mirror conductive wires comprising the first cutting device 38. This configuration still enables the described radiofrequency (RF) arc for cutting tissue between the conductive wires comprising the first cutting device 38 and the conductive wires comprising the second cutting device 58.

According to **FIG. 8**, the metal frame 110 of the body section 114 may be comprised of a wire mesh or woven structure, strut framework, or other stent frame structure, such as a cannula cut stent, that can be radially compressible and expandable. The body section 114 is shown extending axially at a uniform cross-sectional profile or cylindrical profile, although the body section 114 may have an outward or inward curvature or may taper along the axis in the distal direction to have an increasingly larger or smaller cross-sectional area profile along different regions of the body section for better conformity within the body vessel.

The coupling between the distal end 118 of the proximal tapered section 112 and the proximal end 120 of the body section 114 may form a bend or vertex at this transitional region. For example, the ends of the wire members 126 coinciding with the distal end 118 may be coupled individually to the proximal end 120 of the body section 114. The distal end 122 of the body section 114 may include additional connector struts such that when the body section 114 is radially expanded the distal end 122 forms a complete ring or annular shape. In one example, the proximal tapered section 112 and the body section 114 may be formed integrally from a laser cut or chemically etched single cannula made from an electrically conductive material. The metal frame 110 may be constructed from an electrically conductive material, and in particular, the metal frame 110 may be constructed from a degradable metal polymer (e.g., bioresorbable metal material) that degrades safely over a period of time within the human body. The proximal tapered section 112 also may be formed separate from the body section 114 for coupling to the body section 114 by soldering or otherwise attached for electrical communication there between and to withstand the bending and axial forces of the use and the environment.

An electrically insulating material may coat the metal frame 110. Portions of the electrically insulating material may be selectively removed from the metal frame 110 to expose the electrically conductive material of the metal frame 110 in a desired pattern. The desired pattern on the metal frame 110 of the second cutting device 58 that is created by selectively removing the electrically insulating material to expose the electrically conductive material of the metal frame 110, may be configured to mirror and match the desired pattern of the first cutting device 38. For example, the desired pattern created on the metal frame 110 may be configured to mirror and match the desired pattern on the balloon device 210 that comprises the first cutting device 38 according to some embodiments. This configuration is exemplified by the electrically conductive areas 501 exposed on the balloon device 510 (balloon device 510 may be a representation of the second cutting device 58 according to some embodiments) that comprise the desired pattern shown in **FIG. 11****.**

When more than one desired pattern is exposed on the metal frame 110, a same desired pattern, or at least two different desired patterns, may be repeated around a circumference of the metal frame 110. Alternatively, when more than one desired pattern is exposed on the metal frame 110, a same desired pattern, or at least two different desired patterns, may be repeated across multiple locations on the metal frame 110. It follows that the placement of the desired pattern on the metal frame 110 may be a combination of any known pattern of one or more desired patterns.

The wire members 126 may further extend within the second outer sheath 52 and coupled to the wires of the second electrical conductor 20 similarly to what is shown for the first device in **FIG. 2****.** The second electrical conductor 20 may include the appropriate number of wires for transmitting the electrical energy to the conductive material of the second cutting device 58. In another example, the second electrical conductor 20, or an intermediate conductor, may be extended within the second outer sheath 52 and having a contact end for electrically coupling to the electrically conductive material of the second cutting device 58 at one or more locations on the second cutting device 58. This way, the power supply 12 is able to control power transmitted to the second cutting device 58 to electrically energize the electrically conductive material of the second cutting device 58.

**FIG. 9** depicts an example of the second cutting device 58 according to some embodiments where the second cutting device 58 is comprised of an inflatable balloon device 510. The inflatable balloon device 510 depicted in FIG. 9 may include the same, or similar, components as the inflatable balloon device 210 depicted in FIG. 6. An inner cannula 512 of the balloon device 510 is surrounded by a balloon membrane 514 sealably attached to the inner cannula 512 at proximal and distal attachment locations 516, 518. The inner cannula 512 includes one or more longitudinal inflation lumens having a proximal end (not shown) that is capable of communication with a syringe of inflation fluid or the fluid delivery device. The distal end of the inflation lumen is in communication with ports 520, 521 formed into the sidewall of the inner cannula 512. Inflation fluid may be introduced at the proximal end of the second cutting device 58, traversing the inflation lumen and exiting the ports 520, 521 into an inflation annular lumen 522 defined between the inner surface of the balloon membrane 514 and the outer surface of the inner cannula 512 covered and surrounded by the balloon membrane 514. Continued inflation at a suitable pressure causes the balloon membrane 514 to radially expand. In the radially expanded configuration, the balloon membrane 514 may be shaped to have a proximal tapered section 530, a working body section 532, and a distal tapered section 534. The inflation fluid may be an electrically non-conductive to inhibit electrical energy flow into the first device outside the conductive surface. The working body section 532 may define a cylindrical shape as shown or may be tapered for better conformity with the body vessel. Removal of the inflation fluid from the inflation annular lumen 522 at the proximal end of the second cutting device 58 via the inflation lumen causes the balloon membrane 514 to deflate and radially compress into a smaller profile. The second outer sheath 52 may be included for selectively sliding over the balloon membrane 514 when in the deflated configu ration.

A sleeve 540 (shown in dashed lines) may be disposed about the outer surface of the working body section 532 of the balloon device 510. For the working body section 532, the sleeve may wrap around the outside of the working body section 532 and be attached thereto, for example, by an adhesive or other suitable attachment means. The sleeve 540 may comprise of a solid sheet of electrical conductive material or a perforated or otherwise slotted sheet of electrically conductive material in a desired pattern. Alternatively, the sleeve 540 may comprise a solid sheet of electrically conductive material covered by an electrically insulating material, where the electrically insulating material is selectively removed to expose the electrically conductive material in a desired pattern. Alternatively, the sleeve 540 may comprise a solid, perforated, or otherwise slotted polymer or textile sheet attach to the working body section 532 and having an electrically conductive material disposed thereon in a desired pattern. In one example, a pattern of electrically conductive material may be printed or otherwise applied to the outer surface of the working body section 532 of the balloon membrane 514, or to the outer surface and/or inner surface of the sleeve 540 when employed.

According to some embodiments, in addition to or instead of the sleeve 540, the material of the balloon membrane may be comprised of an electrically conductive material, such as a polymer interspersed with the electrically conductive material to create, according to some embodiments, a desired pattern of electrically conductive areas. For the working body section 532, the sleeve 540 may wrap around the outside of the working body section 532 and attach thereto, for example, by an adhesive or soldered. In addition to, or alternatively, to the crisscross pattern shown by the sleeve 540 in **FIG. 9**, **FIG.11** shows another exemplary desired pattern comprised of electrically conductive areas 501 exposed on the balloon device 510 itself. The desired pattern shown in **FIG. 11** includes five distinct electrically conductive areas 501 in the desired pattern. The five distinct electrically conductive areas 501 in the desired pattern of the second cutting device 58 shown by **FIG. 11** may be configured to mirror and match the five distinct electrically conductive areas 201 in the desired pattern of the balloon device 210 embodiment of the first cutting device 38 shown in **FIG. 10****.** As described, the desired pattern of electrically conductive areas 501 may be exposed areas on the sleeve 540, or alternatively printed or otherwise applied to the outer surface of the working body section 532 of the balloon membrane 514, or to the outer surface and/or inner surface of the sleeve 540. Other desired patterns of electrically conductive areas 501 are within the scope of this disclosure.

The electrically conductive material that is applied or printed may be a flowable ink material. The electrically conductive material may be printed on the sleeve 540 or directly on the working body section 532 of the balloon device 510 (e.g., balloon membrane 514) using pad printing or needle ink printing. Factors for selecting suitable electrically conductive materials are the flexibility of the material comprising the balloon membrane 514 and the flexibility of the printed electrically conductive material. The electrically conductive material may be applied to the balloon membrane 514 when the balloon device 510 is in the deflated configuration or the fully or partially inflated configuration.

When more than one desired pattern is included on the balloon device 510, a same desired pattern, or at least two different desired patterns, may be repeated around a circumference of the balloon device 510 (e.g., around a circumference of the working body section 532). Alternatively, when more than one desired pattern is included on the balloon device 510, a same desired pattern, or at least two different desired patterns, may be repeated across multiple locations on the balloon device 510 (e.g., repeated across area of the working body section 532). It follows that the placement of the desired pattern on the balloon device 510 may be a combination of any known pattern of one or more desired patterns.

The second electrical conductor 20 may include the appropriate number of wires for transmitting the electrical energy to the balloon device 510 with the electrically conductive material of the second cutting device 58. The second electrical conductor 20, or an intermediate conductor connecting the second electrical conductor 20 to the balloon device 510, may be extended within the second outer sheath 52 and having a contact end for electrically coupling to the electrically conductive material of the balloon device 510 at one or more locations of the balloon device 510. This way, the power supply 12 is able to control power transmitted to the balloon device 510 to electrically energize each of the electrically conductive areas that comprise the desired pattern formed by the electrically conductive material together at a same time, or control power to each of the electrically conductive areas that comprise the desired pattern separately and individually. When more than one desired pattern is included on the balloon device 510 (e.g., same desired pattern repeated two or more times, or at least two different desired patterns), the power supply 12 is able to control power transmitted to each of the desired patterns together, or individually.

The first device 30 and the second device 50 of the cutting system 10 are operable cooperatively for cutting tissue based on the electrical power or current delivered by the power supply 12 to a contacted, or targeted, portion of tissue within a patient. The tissue primarily contemplated is an aorta dissection flap; however, other applications of the cutting system 10 may include small blood vessels in need of cauterization, tumor, or other undesirable tissue to be removed from the patient. The first device 30 and the second device 50 may be configured to be manipulated by a human operator and/or a robot.

For example, the RF generator (RFG) of the power supply 12 may include a RF energy generator such that electrical current flows to the first cutting device 38 of the first device 30 (or active electrode), and the second cutting device 58 of the second device 50 (or return electrode). The first cutting device 38 and the second cutting device 58 are disposed across from each other on the sides of the tissue to be cut or perforated in engagement with the tissue sides. In particular, the first cutting device 38 and the second cutting device 58 may be disposed on either side of the tissue to be cut or perforated such that the desired pattern on the first cutting device 38 mirrors and matches up to the desired pattern on the second cutting device 58.

An external alignment device may control rotation of the first cutting device 38 and/or the second cutting device 58 to align the desired patterns to mirror and match each other across the tissue. A predetermined alignment position of the desired pattern on the first cutting device 38 may be stored on a memory of the alignment device, and a predetermined alignment position of the desired pattern on the second cutting device 58 may be stored on the memory of the alignment device. This way, the alignment device controls rotation of the first cutting device 38 and the second cutting device 58 until the alignment device determines each of the respective desired patterns are aligned to mirror and match each other across the tissue, based on the predetermined alignment positions. In addition or alternatively, a low level electrical current from the power supply 12 may be alternatively applied to the first cutting device 38 and the second cutting device 58 while the tissue is between them, so that an attraction between the respective desired patterns of the first cutting device 38 and the second cutting device 58 may be detected by the alignment device. The low level electrical current for detecting alignment of the desired patterns is lower than a tissue cutting current described below.

In addition or alternatively, alignment of the desired patterns on the respective first cutting device 38 and second cutting device 58 may be detected based on a set of rulers or markers on the first outer sheath 32 and the second outer sheath 52. The first outer sheath 32 and the second outer sheath 52 may be pre-manufactured to include the rulers (e.g., dash lines representing a length) and/or markers (e.g., visual indicators such as arrows) that are configured to indicate an alignment of the desired patterns on the respective first cutting device 38 and second cutting device 58 to a user handling the first cutting device 38 and the second cutting device 58 from outside.

The RF energy generator may be suitable for a sesquipolar application between the first cutting device 38 and the second cutting device 58, where the second cutting device 58 is larger than the first cutting device 38. Electric current provided by the power supply 12 may be oscillated between the first cutting device 38 and the second cutting device 58. When the first cutting device 38 includes more than one conductive probe 70, or a plurality of desired patterns of electrically conductive material, the energizable tip 72 or desired pattern on the first cutting device 38 may be sequentially energized during power. For example, the energizable tip 72 may remain deployed and axially move along the tissue, while the tip 72 are sequentially phased on and off. In this arrangement, the cuts formed in the tissue are formed one at a time. The selective energization may be controlled by the control switch 24 or there may be a switching module (hardware or software) included with the power supply 12. In another example, the energizable tip 72, or desired patterns of electrically conductive material, may be energized at the same time during oscillation to form more than one cut at a time.

When energized, the smaller surface area of the first cutting device 38 and the larger cross-sectional area of the second cutting device 58 are operable together to form a cut into the tissue by tissue vaporization and RF arc generation, as appreciated by those of skill in the art. The RF arc at the tissue is generated, with first cutting device 38 (e.g., the energizable tip 72, or electrically conductive material in the desired pattern on the first cutting device 38) acting as an active electrode, and the second cutting device 58 (e.g., expandable body 100, or balloon device 510) acting as the return probe, which directs energy at the tissue interface positioned between the first cutting device 38 and the second cutting device 58. In embodiments where the first cutting device 38 is comprised of the energizable tip 72, the RF arc is most intense at the engagement region 81 of the energizable tip 72, which may move toward the second cutting device 58 with exerted pressure. The cutting current may be constant, intermittent or a combination of both. In one example, the cutting current is constant for cutting without coagulation.

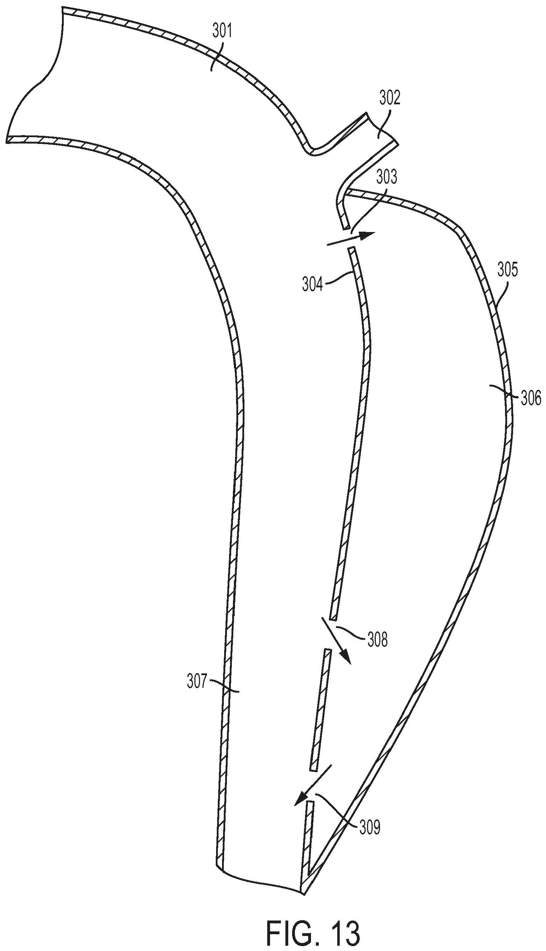

**FIG. 13** illustrates a non-limiting example of a type B dissection in the human aorta. A tear 303 in the inner layer of the body vessel 301, shown in **FIG. 13** as the aorta, distal to the subclavian artery 302 typically allows blood to enter into the aortic wall (see arrows) and separate, or peel, the inner layer 304 of the aorta from the outer layer 305. The space created by the blood between the two layers is referred to as the false lumen 306. The tear 303 is referred as the primary entry point into the false lumen. The separated inner layer 304 is referred to as the flap. The portion of the aorta within the inner layer of the aorta along the dissection is referred to as the true lumen 307. In some cases, multiple entry and exit openings may exist along the flap, as indicated by the flap openings 308 and 309.

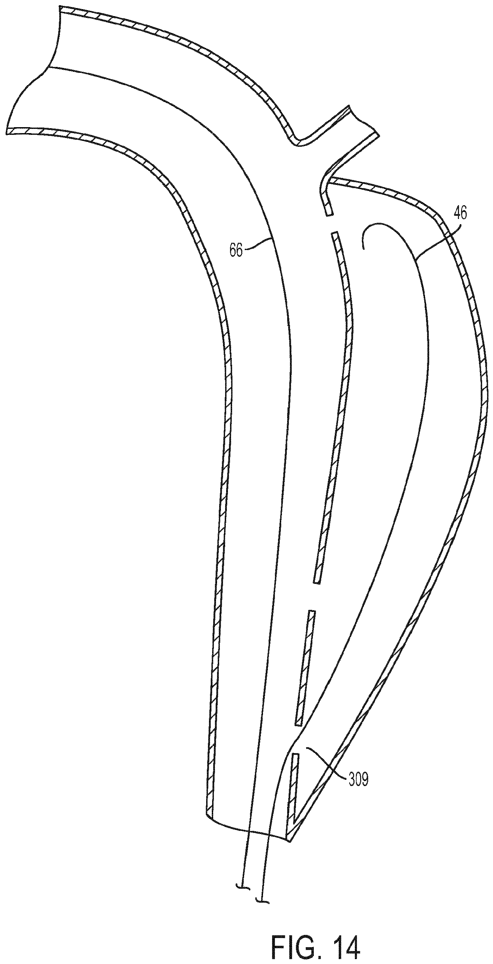

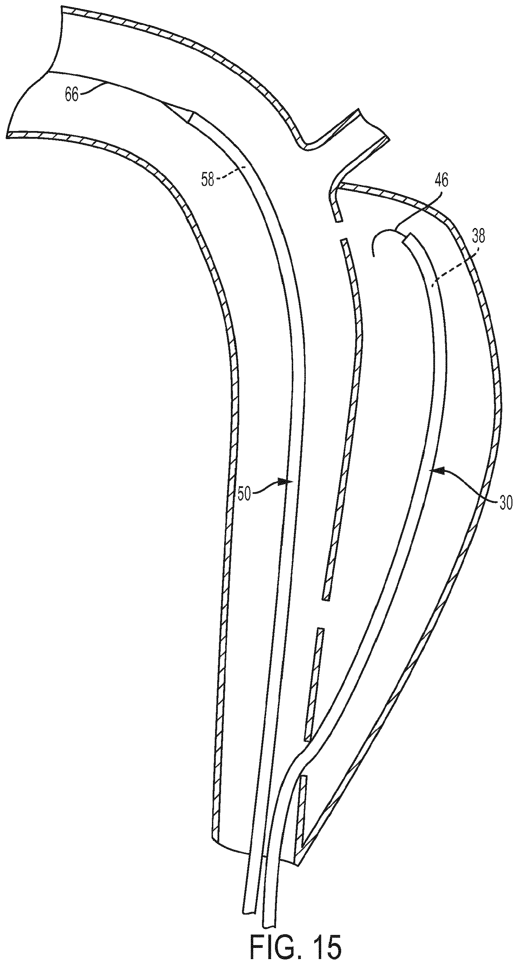

Methods of cutting a tissue will now be described. **FIGS. 14-18** illustrate one application of cutting a tissue, which involves a method of treatment for an aortic dissection with the systems described herein. As may be appreciated by one of ordinary skill in the art, other methods of cutting a tissue are within the scope of this disclosure, such as but not limited to, methods of treating blood vessels in need of cauterization or fistulas, or methods of removing tumors or other undesirable tissue. The order of the steps described below may be altered or adjusted, and some of the steps may be omitted or combined with other steps. **FIG. 14** illustrates the advancement of two guidewires from the femoral artery into the aorta. The first guidewire 46 is located in the first lumen (i.e., false lumen), and the second guidewire 66 is placed into the second lumen (i.e., true lumen). The first guidewire 46 is shown extending through one of the flap openings 309. **FIG. 15** illustrates the second device 50 comprising the second cutting device 58 inserted over the second guidewire 66 to travel into the true lumen, and the first device 30 comprising the first cutting device 38 inserted over the first guidewire 46 to travel into the false lumen. In the next step, the expandable frame 100 of the second device 50 is deployed in the true lumen.

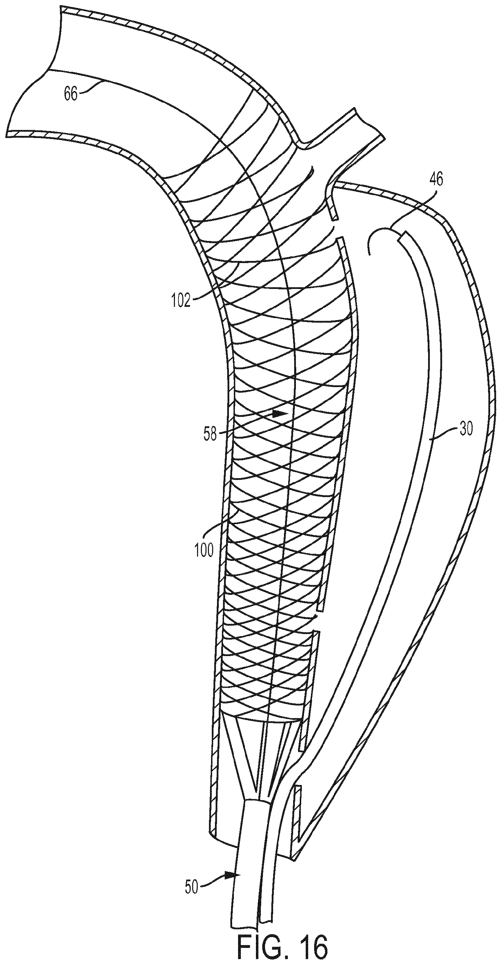

**FIG. 16** illustrates the second cutting device 58 comprising the expandable body 100 with electrically conductive material (such as a metal frame) deployed in the true lumen and the second outer sheath 52 retracted. The radial expansion of the expandable body 100 within the true lumen may cause the volume of the true lumen to expand such that the volume of the false lumen is decreased, thus causing some blood in the false lumen to be displaced out of the false lumen. The inner cannula 60 and the nose cone dilator 65 of the second device 50 are removed for clarity in **FIGS. 16-18****.**

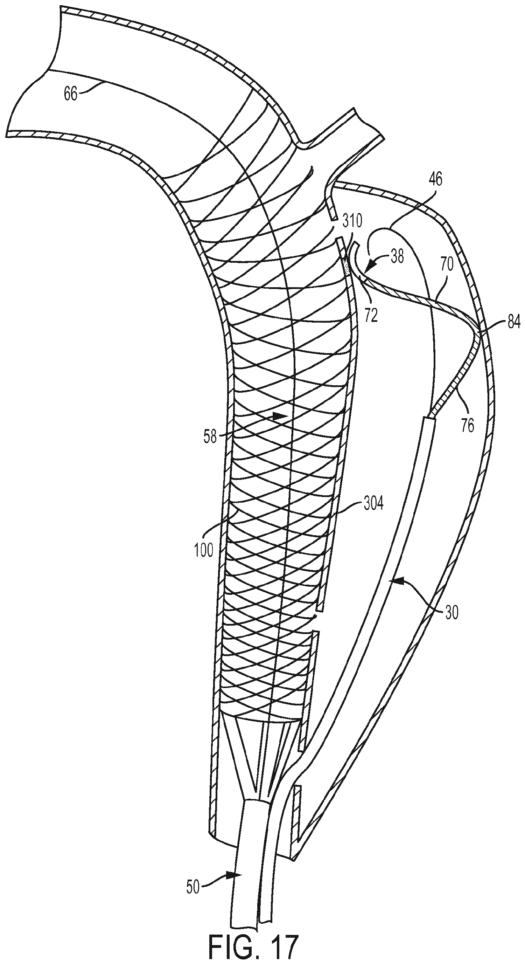

**FIG. 17** illustrates the first cutting device 38 comprising the conductive probes 70 each with the energizable tip 72 unsheathed (partially deployed). The second curved region 76 is configured to be oversized for the size of the false lumen. To this end, engagement of the atraumatic bend 84 along the second curved region 76 with the vessel wall, opposite the separated inner layer 304 (i.e, dissection flap), causes the conductive probe 70 to flex. This flexing causes the energizable tip 72 to move in a direction away from this engagement toward the dissection flap, thereby increasing the downward pressure of engagement of the energizable tip 72 against the dissection flap. The energizable tip 72 and the expandable body 100 with conductive material are on opposite sides of the separated inner layer 304, and in some instances, each applying radial forces against each other to squeeze the separated inner layer 304 between them. The additional downward pressure into the separated inner layer 304 may help form the cuts faster. With additional reference to **FIG. 1**, with the power to the power supply 12 and the RF generator RFG activated, the control switch 24 may be actuated by the operator to provide selective electrical power or current from the power supply 12 to (thereby energizing) the first device 30 and the second device 50. As described previously, electric current may be oscillated between the energizable tip 72 and the expandable body 100 or provided to the energizable tip 72 to cut or perforate the tissue, thereby forming a cut 310, which may be in the form a slit.

When additional conductive probes are present, multiple cuts may be formed by a plurality of energizable tips. In one example, the conductive probes may be deployed together such that the energizable tips engage different regions of the dissection flap. With the probes deployed, the power supply 12 may be configured to phase on and off sequentially the different energizable tips during a cycle. After the cycle, the operator may axially move the energizable tips along the flap without retrieval to or form another row of cuts. Alternatively, the conductive probes may be deployed one at a time, as may be appreciated by one of ordinary skill in the art. The regions may be aligned or may be staggered. In one example, only one energizable tip 72 from the plurality of energizable tips is energized at a single time, thereby forming one cut at a time. In another example, more than one or all of the energizable tips may be energized at a single time, thereby forming a corresponding number of cuts.

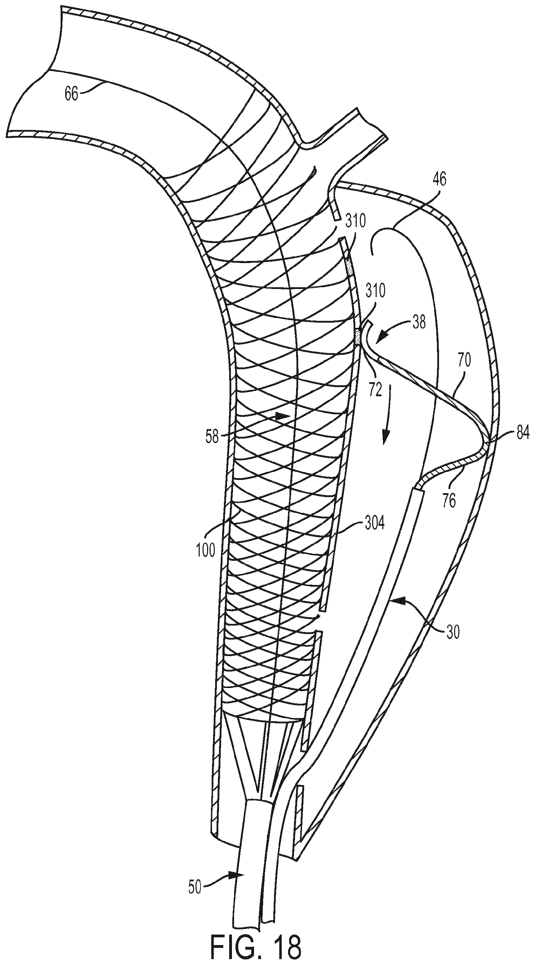

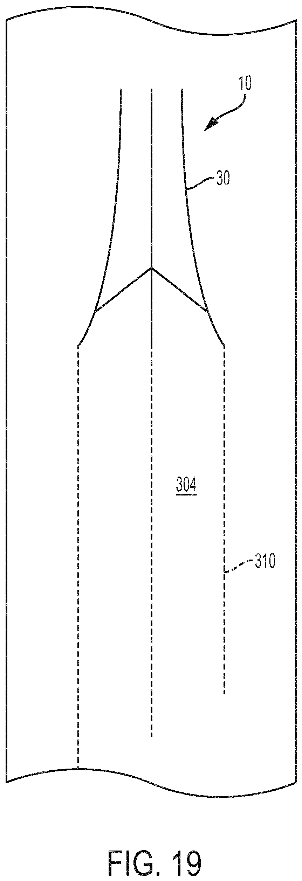

As illustrated in **FIG. 18**, the expandable body 100 of the second cutting device 58 of the second device 50 may be maintained in its position within the body vessel, although the expandable body 100 is retrievable, as described above, for repositioning within the vessel or completely withdrawn from the vessel. After deenergization, the first device 30 is repositioned such that the energizable tip 72 forms additional cuts 310 along a different axial region of the flap. The control switch 24 may be utilized to turn off power to the first device 30 and the second device 50 during repositioning. **FIG. 19** illustrates one pattern of cuts 310 that may be formed into the separated inner layer 304 or flap with the cutting system 10 using the energizable tip 72, or plurality of energizeable tips, as described herein. Different patterns may be formed depending on the number of probes.

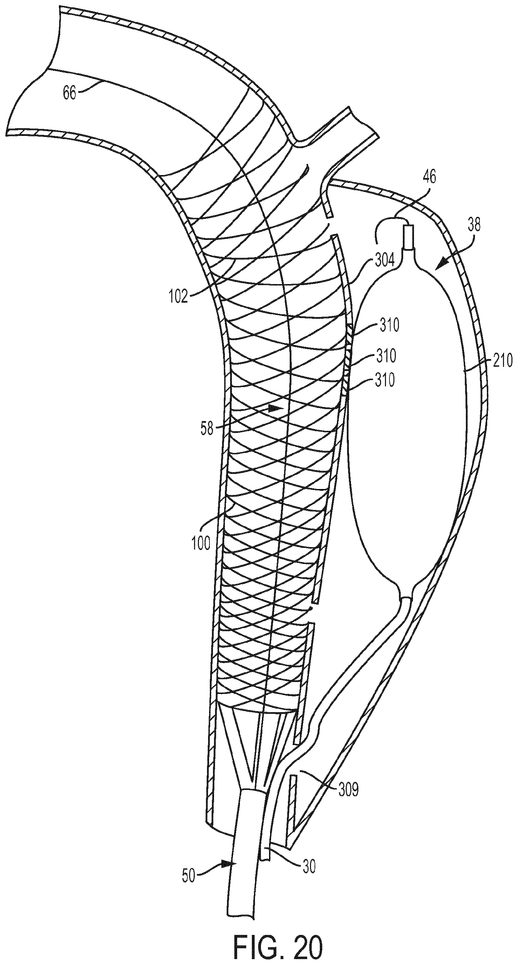

**FIG. 20** shows another application of cutting the inner layer 304 of the aorta tissue, according to another embodiment of the cutting system 10 where the first cutting device 38 is comprised of the balloon device 210, and the second cutting device 58 is comprised of the expandable body 100. The expandable body 100 is comprised of an electrically conductive material (e.g., metal frame) covered in an electrically insulating material, where the electrically insulating material is further selectively exposed in a desired pattern. The desired pattern on the expandable body 100 may correspond to the desired pattern of electrically conductive areas 501 shown in **FIG. 11****.** The balloon device 210 may also have its own desired pattern of exposed electrically conductive material such as, for example, the desired pattern of electrically conductive areas 201 shown in **FIG. 10****.** The desired pattern of electrically conductive areas 201 on the balloon device 210 comprising the first cutting device 38 is configured to mirror and match the desired pattern of electrically conductive areas 501 on the expandable body 100 comprising the second cutting device 58.

**FIG. 20** illustrates the advancement of two guidewires from the femoral artery into the aorta. The first guidewire 46 is located in the first lumen (i.e., false lumen), and the second guidewire 66 is placed into the second lumen (i.e., true lumen). The first guidewire 46 is extended into the false lumen through one of the flap openings 309, where the first device 30 is inserted over the first guidewire to travel into the false lumen. The second guidewire 66 is extended into the true lumen, where the second device 50 is inserted over the first guidewire to travel into the true lumen. **FIG. 20** **further** illustrates the expandable frame 100 of the second device 50 is deployed in the true lumen.

**FIG. 20** illustrates the expandable frame 100 expanded within the true lumen to engage and contact the walls of the true lumen, including the inner layer 304. **FIG. 20** further illustrates the balloon device 210 comprising the first cutting device 38 expanding within the false lumen. To this end, the balloon device 210 may be expanded within the false lumen such that the balloon device engages and contacts both the tissue wall opposite the separated inner layer 304 (i.e, dissection flap), as well as the inner layer 304 itself. The alignment device may further rotate the balloon device 210 and/or the expandable frame 100 such that the desired pattern on each of the balloon device 210 and the expandable frame 100 mirror and match each other through the inner layer 304. This expansion configuration where both the expandable frame 100 and the balloon device 210 are expanded within the true lumen and the false lumen, respectively, offers at least two advantageous results. First, at least some of the blood trapped in the false lumen is evacuated out from the false lumen, which is a desired goal of the cutting application. Second, the expansion configuration decreases an amount of blood remaining between the balloon device 210 and the inner layer 304, and/or amount of blood remaining between the expandable frame 100 and the inner layer 304. By decreasing the amount of blood (or other fluid composition) between the balloon device 210 and the inner layer 304, and the amount of blood (or other fluid composition) between the expandable frame 100 and the inner layer 304, the sesquipolar electrode cutting arrangement between the expandable frame 100 and the balloon device 210 may increase in performance. The increase in performance may translate to faster cuts along the inner layer 304. With additional reference to **FIG. 1**, with the power to the power supply 12 and the RF generator RFG activated, the control switch 24 may be actuated by the operator to provide selective electrical power or current from the power supply 12 to (thereby energizing) the first device 30 and the second device 50. As described previously, electric current may be oscillated between the balloon device 210 and the expandable body 100, or provided to balloon device 210 alone, to cut or perforate the tissue, thereby forming a cut 310, which may be in the form of a slit corresponding to the common desired pattern on the balloon device 210 and the expandable body 100.

Multiple cuts may be performed when the desired pattern includes a plurality of electrically conductive areas, such as the electrically conductive areas 201 and electrically conductive areas 501 included in the desired patterns illustrated in **FIG. 10** and **FIG. 11. FIG. 12** shows the resulting cuts 601 on the tissue from the sesquipolar electrode cutting application using the desired pattern of electrically conductive areas 201 and 501 illustrated in **FIG. 10** and **FIG. 11**, respectively.

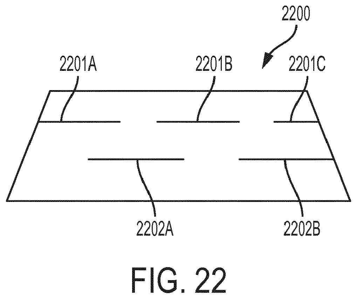

**Fig. 22** shows another exemplary pattern 2200 comprised of a number of first row cuts 2201 A, 2201 B, 2201C and a number of second row cuts 2202A, 2202B. The first row cuts 2201 A, 2201B, 2201C are shown to form a first row, and the second row cuts 2202A, 2202B are shown to form a second row, where the first row cuts 2201A, 2201B, 2201C are positioned to stagger with the second row cuts 2202A, 2202B along a longitudinal direction. Each of the cuts 2201A, 2201B, 2201C and 2202A, 2202B may be shaped as slits having a small width relative to a longitudinal length, where the longitudinal length may be about 1 to 5 cm. In one example, the longitudinal length of one or more of the cuts may be 3 cm. The pattern 2200 (or other patterns described herein) may be repeated along a longitudinal length of the tissue (e.g., the tissue flap separating the true lumen and the false lumen) that does not exceed 40% of the longitudinal length of the tissue. Different patterns may also be formed that follows a specific desired pattern. Any of the patterns described herein may be repeated along the longitudinal length of the tissue up to a predetermined length, up to, and including, the entire length of the tissue.

When the desired pattern is configured to include a plurality of desired patterns, the power supply 12 may be configured to phase on and off the distinct desired patterns sequentially according to a predetermined timing schedule during a cycle. Alternatively, one or more of the desired patterns may be deployed one at a time, or according to a predetermined sequential order, thereby forming a corresponding number of cuts in the inner layer 304.

As illustrated in **FIG. 20**, cuts 310 are made along the inner layer 304 that line up to the desired pattern of electrically conductive areas on the balloon device 210 and the expandable body 100. During a realignment period where the alignment device is moving the balloon device 210 and the expandable body 100 so that their respective desired patterns will mirror and match with each other across the inner layer 304, the control switch 24 may be utilized to turn off power to the first device 30 and the second device 50 during this realignment period.

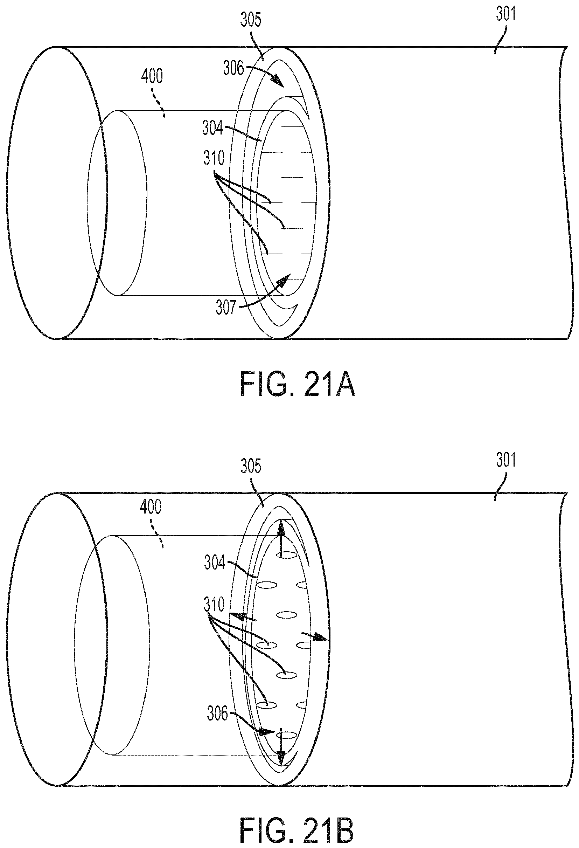

The formation of the cuts 310 at the treatment site may be beneficial in reapposing the separated inner layer 304 or flap to the body vessel 301 or aortic wall. **FIGS. 21A-21B** are illustrations of the body vessel 301 after the usage of the cutting system 10, with another medical treatment device 400 inserted within the body vessel 301. That is, after a suitable number of cuts 310 have been formed into the separated inner layer 304 or flap and the cutting system is removed from the body, other medical treatment devices 400, such as but not limited to, expandable stents (optionally biodegradable), expandable stent grafts (optionally biodegradable), or inflatable balloons, can be inserted in the true lumen 307 of the body vessel 301, as shown in **FIG. 21A****.** These other devices 400 may be located in the true lumen (as shown) of the body vessel 301 with cuts 310 at the treatment site and expanded to reappose the separated inner layer 304 or flap against the wall of the body vessel 301. According to some embodiments, the other devices 400 may also be made from a biodegradable material. **FIG. 21B** depicts the transition of the flap to full reapossition against the vessel wall. The cross-sectional area of the cuts 310 may expand when the flap is reapposed. This reapposition step may allow thrombosing of the false lumen and maintaining patency of the true lumen to reestablish the original flow lumen of the body vessel, such as the aorta, and to facilitate healing of the vessel. The perforated flap may also allow more blood to be squeezed out of the false lumen, thereby avoiding the pooling of blood within the false lumen.

The electrically conductive material described above for any of the components may include platinum, gold, copper and/or silver, or alloys thereof. In one example, the conductive probe may be made of a shape memory alloy such as Nitinol. Other conductive materials may be used without departing from the scope of this disclosure.

Portions of the first device 30 and the second device 50 may be covered in insulation or may be otherwise insulated such that the first and second cutting devices 38, 58 may deliver electrical energy from the power supply 12 to the contacted tissue. The electrical insulation may be any appropriate electrically insulating material including, but not limited to, plastic, rubber, vinyl, epoxy, parylene, or ceramic and may enable the operator to grasp the devices as well as protect the patient's body.

Although examples of the cutting system 10 described herein use sesquipolar RF energy generation for tissue cutting, other cutting systems may be used such as, but not limited to, a bipolar or monopolar RF energy system, a laser system, an ultrasound system, an ultrasonic system, an electrical voltage system, a mechanical blade system, a microwave system, and a cryogenic fluid system.

For a bipolar arrangement, the amount of surface area for the first cutting device 38 and for the second cutting device 58 may be substantially the same. For example, the surface area of the energizable tip 72 of the first cutting device 38 and of the conductive material of the second cutting device 58 may be substantially the same. The expandable body 100 comprising the second cutting device 58 may include a surface area of conductive material disposed, such as, for example, along the sleeve 540 or the balloon membrane 514. Alternatively, the second cutting device 58 may be covered in insulation and have selectively exposed areas that expose the electrically conductive material comprising the second cutting device 58 in a desired pattern.

The power supply 12 may be configured in a manner to generate the RF arc through the bipolar arrangement. With the first cutting device 38 disposed within the false lumen, and the second cutting device 58 disposed within the second lumen of the body vessel, the bipolar arrangement is configured to form cuts within the flap. For a monopolar arrangement, the first cutting device 38 is placed against the tissue within the false lumen. A return electrode pad is attached externally to the patient, so the electrical current flows from the power supply to the first cutting device 38 through the tissue to form cuts within the flap, to the patient return electrode pad and back to the power supply. The cuts formed according to the tissue cutting methods described herein may follow a common desired pattern found on the first cutting device 38 and the second cutting device 58.

While various embodiments have been described, the disclosed features are not to be restricted except in light of the attached claims and their equivalents. Moreover, the advantages described herein are not necessarily the only advantages, and it is not necessarily expected that every embodiment will achieve all of the advantages described.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 62/459,344, from which this application claims priority, and in the abstract accompanying this application are incorporated herein by reference.

## Claims

1. A tissue cutting system comprising:
a first device including:
a conductive member; and
a first port configured to receive a first electrical conductor, wherein the first electrical conductor is coupled to the conductive member;
a second device including:
an expandable member positionable to oppose the conductive member through a tissue wall between the conductive member and the expandable member; and
a second port configured to receive a second electrical conductor, wherein the second electrical conductor is coupled to the expandable member; and
wherein the conductive member and the expandable member are relationally positionable to enable generation of a radio frequency (RF) arc between the conductive member and the expandable member to cut the tissue wall in response to electrical power received by at least one of the conductive member or the expandable member.

2. The tissue cutting system of claim 1, wherein the conductive member comprises:
a first outer sheath with a first outer sheath lumen disposed about a first device axis; and
a first conductive probe slidably disposed within the first outer sheath lumen, the first conductive probe including at least one electrically operable tip.

3. The tissue cutting system of claim 2, wherein, when the first conductive probe, the at least one electrically operable tip of the first conductive probe is disposable outside the first outer sheath lumen and axially movable away from the first device axis.

4. The tissue cutting system of claim 2 or 3, wherein the conductive member further includes a second conductive probe also disposed within the first outer sheath lumen, the second conductive probe including at least one electrically operable tip.

5. The tissue cutting system of claim 4, wherein the at least one electrically operable tip of the second conductive probe is disposable outside the first outer sheath lumen and laterally movable away from the first conductive probe.

6. The tissue cutting system of any of claims 2 to 5, wherein the first conductive probe comprises an elongated body including a curvilinear shaped portion, wherein the at least one electrically operable tip is disposed along the curvilinear shaped portion.

7. The tissue cutting system of claim 6, wherein the curvilinear shaped portion includes a first curved region and a second curved region disposed proximal to the first curved region, wherein the first curved region is disposed radially away from the first device axis in a first radial direction, and the second curved region is disposed radially away from the first device axis in a second radial direction different than the first radial direction.

8. The tissue cutting system of any preceding claim, wherein the second device comprises:
a second outer sheath disposed about a second device axis, such that the expandable member is slidably disposed within the second outer sheath; and
a pattern of an electrically conductive material disposed on an outer surface of the expandable member.

9. The tissue cutting system of claim 8, wherein the expandable member is comprised of an expandable frame, the expandable frame including an outwardly tapered section and a barrel section coupled to the outwardly tapered section, wherein the pattern of the electrically conductive material is disposed along the barrel section.

10. The tissue cutting system of any preceding claim 1, wherein the conductive member comprises:
a balloon device, the balloon device including a first pattern of a first electrically conductive material disposed on an outer surface of the balloon device; and
a first outer sheath disposed about a first device axis, such that the balloon device is slidably disposed within the first outer sheath.

11. The tissue cutting system of claim 10, wherein the expandable member is composed of a second electrically conductive material; and
wherein the second device further comprises:
a layer of an at least partially insulating material disposed on an outer surface of the expandable member to produce a second pattern of the second electrically conductive material exposed on the expandable member, wherein the second pattern mirrors the first pattern and preferably the balloon device is configured to be radially expandable within a first space; and the expandable member is configured to be radially expandable within a second space opposing the first space, the balloon device preferably being configured to radially expand within the first space to abut a wall between the first space and the second space; and the expandable member preferably being configured to radially expand within the second space to abut the wall between the first space and the second space.

12. The tissue cutting system of any preceding claim, wherein a surface area of the expandable member is larger than a surface area of the conductive member.

13. The tissue cutting system of any preceding claim, wherein the at least a portion of the second device is composed of a biosorbable metal material.

14. A tissue cutting system comprising:
a first device including:
a first conductive member; and
a first port configured to receive a first electrical conductor, wherein the first electrical conductor is coupled to the first conductive member;
a second device including:
a second conductive member positionable to oppose the first conductive member through a tissue wall between the first conductive member and the second conductive member; and
a second port configured to receive a second electrical conductor, wherein the second electrical conductor is coupled to the second conductive member; and
wherein the first conductive member and the second conductive member are relationally positionable to enable generation of a radio frequency (RF) arc between the first conductive member and the second conductive member to cut the tissue wall in response to electrical power received by at least one of the first conductive member or the second conductive member.

15. The tissue cutting system of claim 14, wherein the first conductive member comprises:
a first outer sheath with a first outer sheath lumen disposed about a first device axis; and
a first conductive probe slidably disposed within the first outer sheath lumen, the first conductive probe including at least one electrically operable tip.

16. The tissue cutting system of claim 15, wherein the second conductive member comprises:
a second outer sheath with a second outer sheath lumen disposed about a second device axis; and
a second conductive probe slidably disposed within the second outer sheath lumen, the second conductive probe including at least one electrically operable tip.

17. A tissue cutting system comprising:
a first device including:
a first wire guide configured to guide the first device into a first space;
a conductive member; and
a first port configured to receive a first electrical conductor, wherein the first electrical conductor is coupled to the conductive member;
a second device including:
a second wire guide configured to guide the second device into a second space;
an expandable member positionable to oppose the conductive member through a tissue wall between the conductive member and the expandable member; and
a second port configured to receive a second electrical conductor, wherein the second electrical conductor is coupled to the expandable member; and
wherein the conductive member and the expandable member are relationally positionable to enable generation of a radio frequency (RF) arc between the conductive member and the expandable member to cut the tissue wall in response to electrical power received by at least one of the conductive member or the expandable member.

18. The tissue cutting system of claim 17, wherein the conductive member comprises:
a balloon device, the balloon device including a first pattern of a first electrically conductive material; and
a first outer sheath disposed about a first device axis, such that the balloon device is slidably disposed within the first outer sheath; and
wherein the expandable member further includes a second pattern of a second electrically conductive material, wherein the second pattern mirrors the first pattern.
